Europäisches Patentamt

European Patent Office

Office européen des brevets

(11) Publication number: 0 661 380 A2

## EUROPEAN PATENT APPLICATION

(21) Application number: 94120312.7

(51) Int. Cl.6: C12M 1/36, C12P 13/14, C12N 1/20

(22) Date of filing: 21.12.94

(30) Priority: 28.12.93 JP 349244/93

(43) Date of publication of application:
05.07.95 Bulletin 95/27

(84) Designated Contracting States:
DE FR GB IT

(71) Applicant: Ajinomoto Co., Inc.
No. 15-1, Kyobashi 1-chome,
Chuo-ku
Tokyo 104 (JP)

(72) Inventor: Nakayama, Tatsuya, c/o Technology
& Eng. Lab.
Ajinomoto Co., Inc.,
1-1 Suzuki-cho,
Kawasaki-ku
Kawasaki-shi,
Kanagawa-ken (JP)
Inventor: Katayama, Masaki, c/o Technology &
Eng. Lab.
Ajinomoto Co., Inc.,
1-1 Suzuki-cho,
Kawasaki-ku
Kawasaki-shi,
Kanagawa-ken (JP)
Inventor: Miyashiro, Shigeyoshi, c/o
Technology & Eng. Lab.
Ajinomoto Co., Inc.,
1-1 Suzuki-cho,

Kawasaki-ku
Kawasaki-shi,
Kanagawa-ken (JP)
Inventor: Okayasu, Sachio, c/o Technology &
Eng. Lab.
Ajinomoto Co., Inc.,
1-1 Suzuki-cho,
Kawasaki-ku
Kawasaki-shi,
Kanagawa-ken (JP)
Inventor: Takahashi, Hiroyasu, c/o
Technology & Eng. Lab.
Ajinomoto Co., Inc.,
1-1 Suzuki-cho,
Kawasaki-ku
Kawasaki-shi,
Kanagawa-ken (JP)
Inventor: Shimizu, Eiko, c/o Technology &
Eng. Lab.
Ajinomoto Co., Inc.,
1-1 Suzuki-cho,
Kawasaki-ku
Kawasaki-shi,
Kanagawa-ken (JP)

(74) Representative: Strehl Schübel-Hopf Groening
& Partner
Maximilianstrasse 54
D-80538 München (DE)

(54) A cultivation method and apparatus for aerobic culture of microorganism.

(57) The growth state of the microorganism in a fermentation for the aerobic culture of a microorganism is controlled to keep it at a suitable level.

This is achieved by a method for culturing microorganisms aerobically which comprises monitoring the growth state of microorganism in a fermenter by measuring directly and simultaneously the concentration of the microorganism cells, the concentration of the carbon source, the concentration of ammonium ion and the concentration of a desired product in the culture broth in the said fermenter with a single measuring means and controlling automatically the temperature, the concentration of the carbon source and the concentration of ammonium ion on the basis of the measured values.

EP 0 661 380 A2

## BACKGROUND OF THE INVENTION

### Technical Field of the Invention

The present invention relates to a method for maintaining a suitable growth of a microorganism by controlling the temperature in an aerobic culture of the microorganism, a method for controlling automatically the concentration of the carbon source and the concentration of ammonium ion to keep the fermentation conditions suitable for the growth of the microorganism and an apparatus suitable for carrying out such a method.

### Description of the Prior Art

The following is a typical culturing procedure in the biochemical industry. Raw materials are fed into a seed fermenter and sterilized by boiling, and then the microorganism cells which were previously cultured germ-free in a flask are inoculated and grown. Culture in a main fermented is prepared as soon as the seed culture is initiated. The inoculated microorganism cells are grown for a certain period of time and fed into the main fermented when it is clearly judged on account of the number of the microorganism cells that the microorganism cells are in the logarithmic growth phase.

The fermentation process in a main fermenter consists of two stages. The first half stage is to mainly increase the microorganism cells and the second one is to mainly produce a desired product. Items necessary for controlling fermentation processes are the adjustment of the culture medium, moreover the feed rate of the carbon source, the temperature, pH, ventilation and so on (Principles of Fermentation Technology: P.F. Stanbury, A. Whitaker; Pergamon Press, 1986). After a certain time, the fermentation is terminated and the desired product is sent to isolation and purification processes.

As a method for controlling the concentration of the carbon source in the culture broth, the controlling method by estimating the concentration of the carbon source in the culture broth from the change of the respiratory activity or the change of the speed of consuming ammonia is known. However, as the concentration of the microorganism cells and the consumption rate of the carbon source were not directly measured, it was difficult to control the concentration of the carbon source to be a certain value due to the occasional change in the metabolic activity of the microorganism.

It is known to be possible to control the growth of microorganism cells by estimating the concentration of the microorganism cells from the turbidity of the culture broth and then controlling the temperature of the culture broth.

However, as the concentration of the microorganism cells is estimated based on the turbidity of the culture broth, a correct estimation of the concentration of the microorganism cells in the culture broth is impossible on account of lot differences of raw materials or admixture of impurities.

Accordingly, it was difficult to control automatically and optimally the temperature of the fermentation broth throughout the culture period.

## OBJECTS OF THE INVENTION

In prior-art methods for controlling fermentation, sampling was necessary to measure the concentration of the microorganism cells, the concentration of the carbon source, the concentration of ammonium ion, the concentration of the desired product and so on in the culture broth of the fermenter.

Since it took a maximum of about an hour to analyze them after sampling, it was difficult to timely and correctly monitor the growth state of the microorganism cells in the fermenter.

The object of the present invention is the development of a method and an apparatus for monitoring correctly the growth state of the microorganism cells in a fermenter in an on-line system by measuring the concentration of microorganism cells, the concentration of the carbon source, the concentration of ammonium ion and the concentration of the desired product in the fermenter, maintaining the suitable growth of the microorganism cells on the basis of the measured values and controlling automatically the concentration of the carbon source and the concentration of ammonium ion in the culture broth throughout the culture period, in the aerobic fed-batch culture.

## BRIEF DESCRIPTION OF THE DRAWINGS

Fig. 1 represents the apparatus used in Examples 1 and 2.

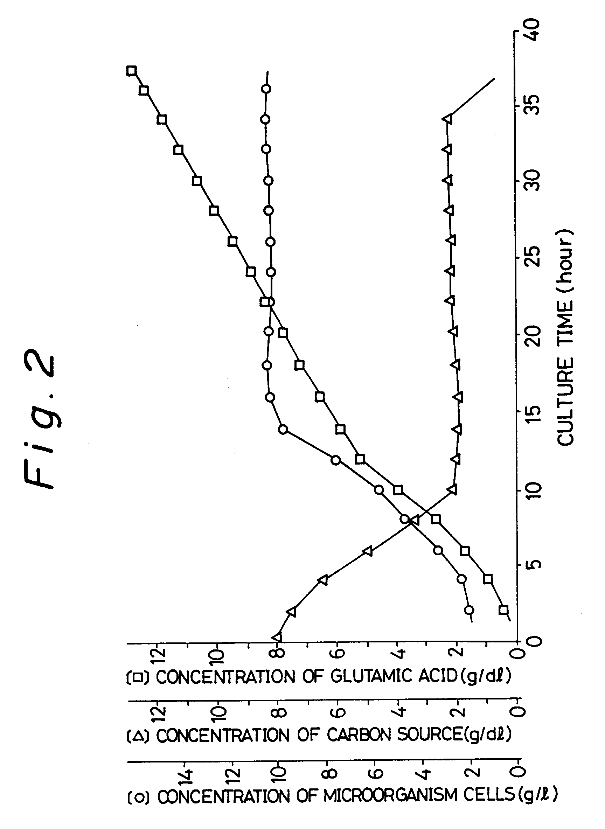

Fig. 2 represents the analysis results of each state in the fermenter in Example 1.

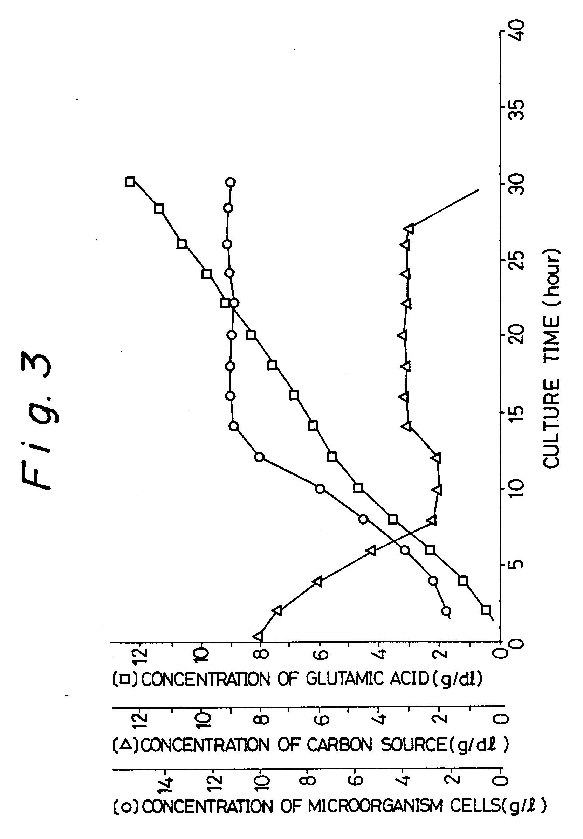

Fig. 3 represents the analysis results of each state in the fermenter in Example 2.
Fig. 4 represents the apparatus used in the Comparative Example.

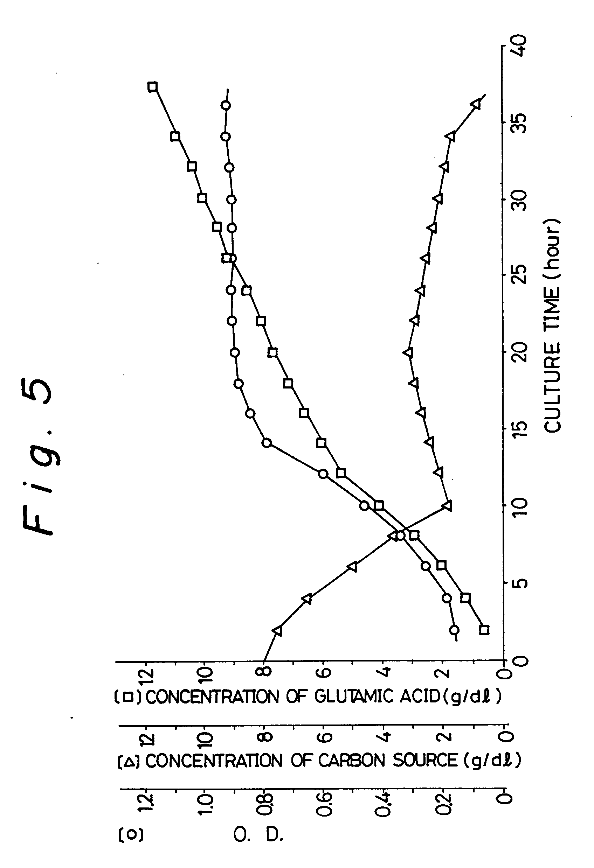

Fig. 5 represents the analysis results of each state in the fermenter in the Comparative Example.

DETAILED DESCRIPTION OF THE INVENTION

The present invention for attaining the said object relates to a method for culturing a microorganism aerobically and an apparatus therefor which are characterized by monitoring the growth state of the microorganism in a fermentation broth in a fermenter by measuring directly and simultaneously the concentration of the microorganism cells, the concentration of the carbon source, the concentration of ammonium ion and the concentration of the desired product in the said fermentation broth in the said fermenter with a single measuring means, and controlling automatically the temperature, the concentration of carbon source and the concentration of ammonium ion in the said fermentation broth on the basis of the measured values.

In the apparatus used for practicing the method of the present invention, the culture broth is sent from the fermenter to a near-infrared spectrometer through a sampling apparatus which is in an on-line system, and is analyzed. It is possible to measure simultaneously the concentration of the carbon source, the concentration of the microorganism cells, the concentration of the desired product and the concentration of ammonium ion with a near-infrared spectrometer. The near-infrared spectrometer is connected to a personal computer and each measured value is transmitted to the personal computer.

Then, the growth state of the microorganism cells in the fermenter is correctly seized on the basis of a single measured value or a plurality thereof. On the basis of the growth state of the microorganism, the temperature of the culture broth is changed and the growth state of the microorganism cells is controlled in a range suitable for increasing the yield of the desired product. The set values of the feed rate of the raw materials to the fermenter are automatically calculated on the basis of the concentration of the carbon source in the culture broth and the control devices of the feed rate are controlled on the basis of the set values. Each set value are sent to the corresponding control device connected to the fermenter through a signal converter attached to the personal computer.

A method for controlling the culture conditions in the present invention is described below.

A summary of the method for the culturing of microorganisms is described as follows:

Microorganism cells for seed culture are added to the initial medium. Then the growth rate of the microorganism cells increases gradually and reaches the maximum rate (the logarithmic growth phase). The growth stops and remains in a so-called stationary state (the stationary phase). Moreover, it reaches a period in which the number of living microorganism cells decreases (the death phase). When a certain amount of the carbon source has been consumed, the culture medium is fed continuously or intermittently (the feed culture phase).

At this time, items necessary for the process control are the adjustment of the culture medium, moreover the temperature, the rate of adding carbon source, pH, ventilation and so on (Biseibutsu Kogaku no Oyo: Shichiji Saburo; Kyoritsu Shuppan Kabushiki Kaisha, P.366-P.381, 1981).

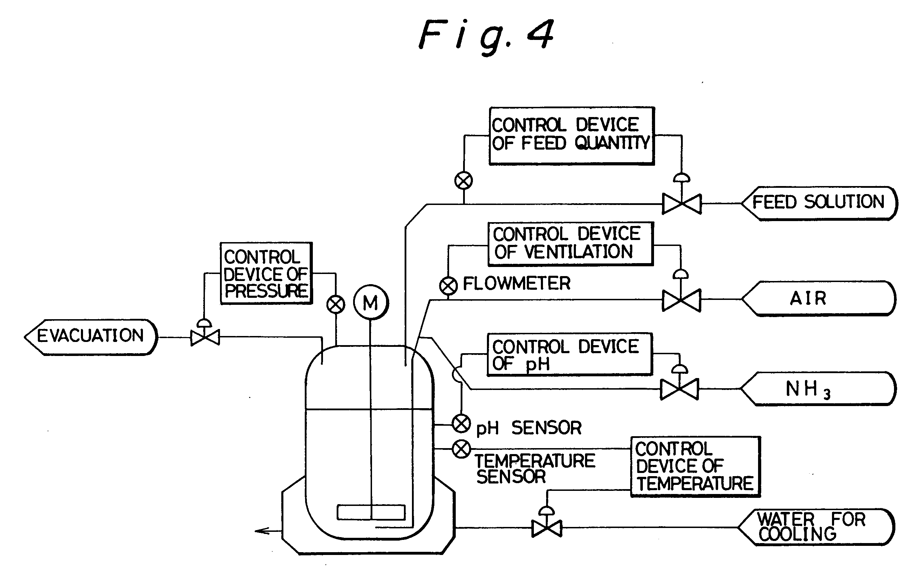

In a prior-art apparatus for cultivation, each control device functions individually on the basis of each measured value as shown in Fig. 4.

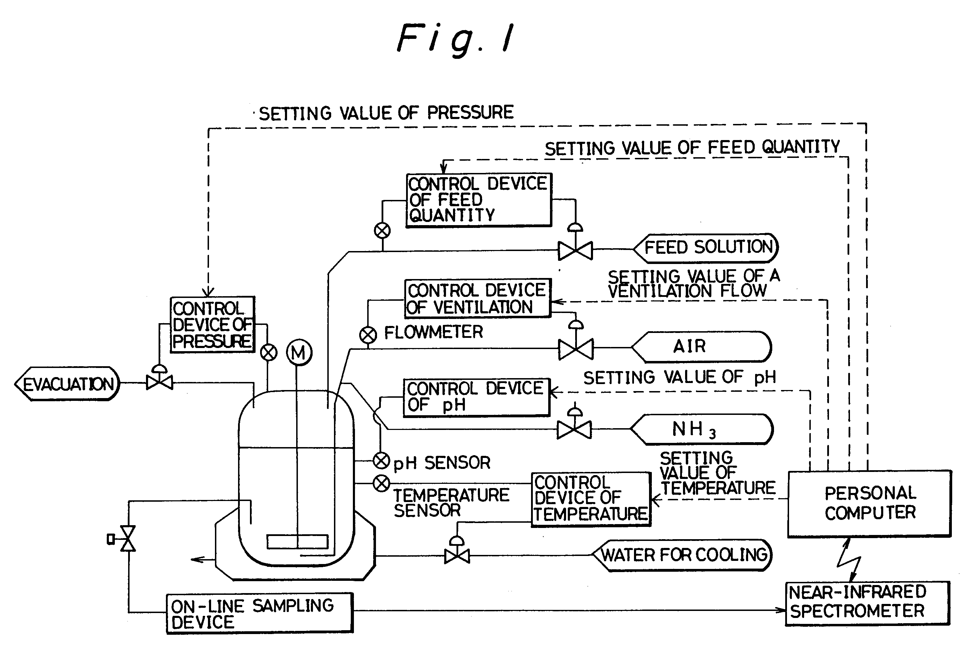

In the apparatus for cultivation used in the present invention, a fermentation broth is analyzed via an on-line system by a near-infrared spectrometer through the on-line sampling device as shown in Fig. 1. The apparatus for the cultivation is provided with a control device of feed rate of the carbon source, a control device of flow rate of aeration for supplying oxygen, a control device of pressure, a control device of pH with an electrode for detecting the pH of the fermentation broth and a control device of temperature with a thermometer for detecting the temperature of the fermentation broth. Each control device implements the directives instructed by a personal computer. The action of each control device is decided by the personal computer on the basis of the analysis values of the near-infrared spectrometer.

In the apparatus of the constitution shown in Fig. 1, the culture is initiated. After a certain period of time as previously input into the personal computer has passed, samples are taken from the fermenter at regular time intervals and the analysis of the components is carried out by the near-infrared spectrometer, wherein regular time intervals, which are the sampling periods, are set without taking the metabolic state or the growth state of the microorganism cells into consideration.

The first feeding in the feed culture phase is initiated when the analysis by the near-infrared spectrometer shows that the concentration of the carbon source in the culture solution of main fermenter before the feed culture becomes lower than a previously set controlling the target value.

3

The feed set value at this time is automatically calculated by the personal computer on the basis of the consumption rate of the carbon source by the microorganism cells and the concentration of the carbon source in the fermentation broth at the start of feeding.

The calculating algorithm of the feed set value is as follows.

The consumption rate of the carbon source in the fermentation broth is calculated based on the analytical values of the concentration of the carbon source at the preceding sampling and at the present sampling, and the quantity of the carbon source in the feed solution added after the preceding sampling. Moreover, the feeding rate of the feed solution is calculated from the dimensional relation between the actual value and the setting value (which is called SV below) of the concentration of residual sugar.

Examples of control methods are as follows.

1) In the case that (the analysis result) > (SV) + 0.2 (g/dl), (the feeding rate) = 0.8×(the consumption rate of the carbon source in the fermentation broth)

2) In the case that (SV) + 0.2 (g/dl) ≧ (the analysis result) > (SV) + 0.1 (g/dl), (the adding rate) = 0.9 × - (the rate of consumption of the carbon source in the fermentation vat)

3) In the case that (the analysis result) = (SV), (the feeding rate) = (the consumption rate of the carbon source in the fermentation broth)

4) In the case that the (SV) - 0.1 (g/dl) > (the analysis result) ≧ (SV) - 0.2 (g/dl), (the feeding rate) = 1.1 × (the consumption rate of the carbon source in the fermentation broth)

5) In the case that the (SV) - 0.2 (g/dl) > (the analysis result), (the feeding rate) = 1.2 × (the consumption rate of the carbon source in the fermentation broth).

However, in the case that the rate at which the desired product is produced is high and the growth of the microorganism cells is within an ordinary range(that is, the metabolic activity of the microorganism cells is high), the culture is carried out at a high concentration of the carbon source for accelerating the consumption rate of the carbon source to shorten the culturing period and increase the productivity.

The temperature of the culture solution is decided by estimating the growth state of the microorganism cells. The growth state of the microorganism cells is estimated by comparing the analytical values of the concentration of the microorganism cells with the range of the concentration of the cells determined previously on the basis of the optimal concentration of the cells in the fermentation in which the high yield was achieved.

The first change of the temperature is automatically made at the time when the growth of the microorganism cells proceeds and the concentration of the microorganism cells reaches a certain level. The range within which the temperature is changed is decided by estimating the growth rate of the microorganism.

Moreover, the second change is made at the time when the concentration of the microorganism cells reaches the level which was set previously. The range within which the temperature is changed is decided by estimating the growth rate of the microorganism like in the first change.

It is possible to control automatically the temperature throughout the fermentation period by repeating the above-mentioned operation and deciding previously the target value for controlling the optimal temperature corresponding to the range of the concentration of the microorganism cells.

In a culture which requires a nitrogen source as a raw material, as it is possible to measure the concentration of ammonium ion directly with a near-infrared spectrometer according to the present invention, and the target value for controlling the feeding rate of the raw material can be automatically calculated.

The target values for controlling the pH in the fermentation broth, the flow rate of the aeration and the pressure in the fermenter are usually kept at certain values.

APPLICABILITY OF THE INVENTION

By the method and the apparatus of the present invention, the concentration of carbon source in the culture broth is directly measured and the target value for controlling the feed rate is set. Accordingly, the concentration of the carbon source in the culture broth can be automatically controlled at a constant level even if the metabolic activity of the microorganism cells varies largely during the culture period.

As the concentration of the microorganism cells and the production rate of the desired product in the culture broth can be directly measured, it is possible that the growth state of the microorganism cells in the culture broth is correctly monitored in spite of the lot difference of the raw materials or the admixture of impurities. Moreover, it is possible that the growth state of the microorganism cells is suitably maintained throughout the culture period by automatically changing the temperature of the culture broth. In addition, as the growth state of the microorganism cells in the culture broth is correctly monitored, the culture conditions are maintained at suitable values. These are all connected to an increase of the productivity. As it is possible to measure the concentration of ammonium ion directly, the target value for controlling the feed rate of the raw materials can be automatically calculated as well.

Examples:

The present invention is further illustrated by the following examples.

INFRA ANALYZER 600 made by BRAN + LUBBE Co. was used as a near-infrared spectrometer. Calibration curves for each of the concentrations of the carbon source, of the glutamic acid, of the microorganism cells and of ammonium ion are described below. The sign F [nnnn] as shown on the calibration curves means the absorbance at the wave-length nnnn (nm).

(The concentration of the carbon source) = 32.36 + 0.900 $\times$ (295.2 $\times$ F[2270] - 1019.0 $\times$ F[2180] - 163.7 $\times$ F[1982] + 848.3 $\times$ F[2100])

(The concentration of the glutamic acid) = -4.064 + 1.079 $\times$ (-383.2 $\times$ F[2190] + 1233.0 $\times$ F[2139] + 122.9 $\times$ F[1982] - 998.5 $\times$ F[2100])

(The concentration of the microorganism cells) = -1.570 + 0.321$\times$ (-100.1$\times$F[2348] + 71.18$\times$F[2208] + 53.83$\times$ F[1445])

(The concentration of ammonium ion) = -2.817 + 0.856$\times$ (-846.5$\times$F[1818] + 878.6$\times$F[1778] - 15.8$\times$ F-[2100])

**Example 1 :**

(Production of glutamic acid by fermentation)

Brevibacterium lactofermentum ATCC 13969 was inoculated into the culture medium shown in Table 1 and a pre-culture was made at 31.5°C for 17 hours.

Table 1

| Components of the culture medium | |
|---|---|
| Components | Concentration |
| Glucose | 3.0 g/dl |
| KH$_2$PO$_4$ | 0.1 g/dl |
| MgSO$_4 \bullet$7H$_2$O | 0.04 g/dl |
| FeSO$_4 \bullet$4H$_2$O | 0.001 g/dl |
| Meat Extract | 1.0 g/dl |
| Urea | 1.4 g/dl |
| MnSO$_4 \bullet$7H$_2$O | 0.001 mg/dl |
| Soybean protein hydrolyzed solution (T-N. 4 g/dl) | 0.098 mg/dl |
| Vitamin B1-HCl | 200 $\gamma$/ℓ |
| Biotin | 300 $\gamma$/ℓ |
| Af (AZ-20R) | 0.0005 mg/dl |

After the pre-culture described above, a culture was continuously carried out in the main fermenter with a capacity of 5 kℓ.

The culture medium is described in Table 2. Other culture conditions are listed in Table 3.

Table 2

| Components of the culture medium | |
|---|---|
| Components | Concentration |
| Carbon source (converted to sucrose) | 8.0 g/dl |
| $KH_2PO_4$ | 0.2 g/dl |
| $MgSO_4 \cdot 7H_2O$ | 0.1 g/dl |
| $FeSO_4 \cdot 4H_2O$ | 2.0 g/dl |
| Thiamine hydrochloride | 200 $\mu$g/$\ell$ |
| Soybean protein hydrolyzed solution (T-N. 4 g/dl) | 2.5 ml |
| Note) Cane molasses and starch saccharification solution (in a ratio of 1:1) were used as the carbon source. | |

Table 3

| Conditions of culture | |
|---|---|
| Quantity of charged solution | 1.5 K$\ell$ |
| Temperature of culture | 31.5 °C |
| Stirring rotation number | 145 rpm |
| pH | 7.8 |
| Culture period | 37 hours |

At the same time when main culture was initiated the culture broth was passed from the fermenter to the near-infrared spectrometer through the on-line sampling device and analyzed.

The sampling period was set at intervals of 10 minutes.

The growth state of the microorganism cells and the concentration of the carbon source were monitored by the personal computer, and each set value for the feed rate of the raw materials to the fermenter and for the temperature was calculated. Each calculated set value was sent to each corresponding control device of the fermenter via the signal converter of the personal computer.

The method for controlling the concentration of the carbon source is as follows.

The carbon source was fed as the feed solution when the concentration of the carbon source contained in the initial culture medium fell below 2 g/dl. The program was previously prepared in the personal computer, which maintains the concentration of the residual sugar at a certain value by controlling the feed rate.

After the initiation of the fermentation, the concentration of the carbon source began to fall. After about 10 hours, the concentration of the residual sugar fell below 2 g/dl according to the analytical values obtained by the near-infrared spectrometer. The feed rate was calculated by the personal computer.

The calculating method is as follows.

The consumption rate of the carbon source in the fermentation broth was calculated based on the analytical values of the concentration of the carbon source at the preceding sampling and at the present sampling, and the quantity of the carbon source in the feed solution added after the preceding sampling. Moreover, the feeding rate of the feed solution was calculated from the dimensional relation between the actual value and the setting value (which is called SV below) of the concentration of the residual sugar. The controlling methods are as follows.

1) In the case that (the analysis result) > (SV) + 0.1 (g/dl), (the feeding rate) = 0.8 × (the consumption rate of the carbon source in the fermenter)

2) In the case that (SV) + 0.1 (g/dl) ≧ (the analysis result) > (SV) + 0.05 (g/dl), (the feeding rate) = 0.9 × (the consumption rate of the carbon source in the fermenter)

3) In the case that (SV) + 0.05 (g/dl) ≧ (the analysis result) > (SV), (the feeding rate) = 0.95 × (the consump tion rate of the carbon source in the fermenter)

4) In the case that (the analysis result) = (SV), (the feeding rate) = (the consumption rate of the carbon source in the fermenter)

5) In the case that (SV) > (the analysis result) ≥ (SV) - 0.05 (g/dl), (the feeding rate) = 1.05 × (the consumption rate of the carbon source in the fermenter)

6) In the case that (SV) - 0.05 (g/dl) > (the analysis result) ≥ (SV) - 0.1 (g/dl), (the feeding rate) = 1.1 × - (the consumption rate of the carbon source in the fermenter)

7) In the case that (SV) - 0.1 (g/dl) > (the analysis result), (the feeding rate) = 1.2 × (the consumption rate of the carbon source in the fermenter)

A concrete example of the calculation is shown below.

In the case that SV at the present time = 2 (g/dl), the analysis result = 1.92 (g/dl), the consumption rate of the carbon source = 20 (Kg/hr), this example corresponds to the condition of the item (6) above and the feeding rate becomes 22 (Kg/hr).

Thereafter, the set value for the feeding rate is calculated repeatedly for each sampling period. As a result of controlling the concentration of the residual sugar under the above-mentioned conditions, the concentration of the residual sugar could be stably kept at 2±0.2 g/dl.

The set value for the control of the temperature of the culture broth was decided as follows. At the time when the culture was initiated the temperature was invariably kept at 31.5°C.

At the time when the growth of the microorganism was proceeding and the concentration of the microorganism cells reached 5.6 g/ℓ, the first change of the temperature was automatically made. As the growth of the microorganism lay within the ordinary range, the temperature was changed to 35.0°C. Moreover, at the time when the concentration of the microorganism cells reached 8.0 g/ℓ, the second change of the temperature was automatically made. As the growth of the microorganism lay within the ordinary range, the temperature was changed to 39.0°C.

After the culture was initiated, polyoxyethylene sorbitan monopalmitate(0.18 g/dl) was automatically added to retard the growth of the microorganism and produce glutamic acid at the time when the concentration of the microorganism cells reached 5.6 g/dl.

It was possible to control the culture condition automatically throughout the culture period by controlling, as said above, the residual sugar as well as the temperature. The concentration of glutamic acid accumulated was 2.5 g/dl and the yield was 56.5%.

**Example 2 :**

(Production of glutamic acid by Fermentation)

Brevibacterium lactofermentum ATCC 13969 was inoculated into the culture medium shown in Table 1 and pre-cultured at 31.5°C for 17 hours. After the pre-culture as described above, a culture was continuously made in the main fermenter with a capacity of 5 kℓ. The culture medium is shown in Table 2 and is the same as that in Example 1. Other culture conditions used are those shown in Table 3 which are same as those in Example 1. The culture was made under the same conditions as in Example 1 in terms of the apparatus and the sampling period.

In the method for controlling the concentration of the carbon source, the carbon source was added as the feed solution when the concentration of the carbon source contained in the initial culture medium had fallen below 2 g/dl. The program was previously prepared for the personal computer, which kept the concentration of the residual sugar at a certain value by controlling the feeding rate. After the initiation of the culture, the concentration of the carbon source began to fall. After about 8 hours and 10 minutes the concentration of residual sugar fell below 2 g/dl according to the analysis results provided by the near-infrared spectrometer. The feeding rate was calculated by the personal computer.

The calculating method is as follows.

The consumption rate of the carbon source in the fermentation broth was calculated based on the analytical values of the concentration of the carbon source at the preceding sampling and at the present sampling, and the quantity of the carbon source in the feed solution added after the preceding sampling. Moreover, the feeding rate of the feed solution was calculated from the dimensional relation between the actual value and the setting value (which is called SV below) of the concentration of the residual sugar.

The controlling methods are the same as in Example 1.

It was recognized however, that the metabolic activity of the microorganism was high since the production rate of glutamic acid was faster than in ordinary fermentations within 13 hours after the initiation of the culture and the growth of the microorganism was within the usual range of ordinary fermentations.

Then the concentration of the carbon source was set higher, namely from 2 g/dl to 3 g/dl, to accelerate the consumption of the carbon source and the culture was initiated to shorten the culture period and increase the productivity. As a result of the control of the concentration of the residual sugar under the above-mentioned condition, the concentration of the residual sugar could be kept at ±0.2 g/dl to the set value.

The set value for the control of the temperature of the culture broth was decided as follows. At the time when the culture was initiated, the temperature was invariably kept at 31.5°C. At the time when the growth of the microorganism proceeded and the concentration of the microorganism cells reached 5.6 g/ℓ within 8 hours after the initiation of the culture, the first change of the temperature was automatically made. As the growth of the microorganism was somewhat faster than ordinarily, the temperature was changed to 36°C to retard the growth of the microorganism. Moreover, at the time when the concentration of the microorganism cells reached 8.0 g/ℓ the second change of the temperature was automatically made. As the growth of the microorganism lay within the ordinary range, the temperature was changed to 39.0°C.

After the culture was initiated, polyoxyethylene sorbitan monopalmitate(0.18 g/dl) was automatically added to retard the growth of the microorganism and produce glutamic acid at the time when the concentration of the microorganism cells reached 5.6 g/dl.

It was possible to control the culture condition automatically throughout the culture period by controlling as mentioned above the residual sugar as well as the temperature. As a result of the culture for 30 hours, the concentration of glutamic acid accumulated was 12.3 g/dl and the yield was 55.5%.

A comparative Example is illustrated below.

Comparative Example :

(Production of glutamic acid by fermentation)

A comparative Example was made under the same conditions as in the Examples regarding both the culture medium and strain.

Concerning the control of the culture conditions, the setting values of controlling the feeding rate, the pH and the temperature were decided and set following manual analysis of the sample and human decision. The sampling and analysis period were set to be 1 hour.

The concentration of the residual sugar was decided based on the analysis result of the sample and by Lehmann-Shuer's analyzer. In the method of controlling the concentration of the residual sugar, the carbon source was added as the feed solution after the concentration of carbon source contained in the initial culture medium had fallen below 2 g/dl. The feeding rate was decided by human decision. As a result of controlling, the concentration of the residual sugar was mainly controlled within the range of 2±1.0 g/dl after the control was stated.

The pH in the culturing was controlled to be kept at 7.8 invariably after the initiation of the culture.

The set value of controlling the temperature of the culture broth was decided as follows. The temperature was kept at 31.5°C at the time when culture was initiated. The turbidity of the 26-fold diluted solution was measured at the time of sampling. When the change of the turbidity reached 0.45, the temperature was set to 35°C as the first change. Then, after 2 hours, the temperature was set to 39°C as the second change.

After the culture was initiated, polyoxyethylene sorbitan monopalmitate(0.18 g/dl) was added to retard the growth of the microorganism and to produce glutamic acid when the change of the turbidity of the 26-fold diluted solution of the sample reached 0.45. As the result of said culture the accumulated concentration of glutamic acid was 11.4 g/dl and the yield was 51.5%. The yield was decreased by 5% compared with the yield of the above Examples.

Also in the fermentation of other amino acids such as L-lysine, L-valine, L-glutamic acid and so on or in the fermentation of the nucleic acid such as inosine, inosinic acid, guanosine, guanylic acid and so on, a similar yield-increasing effect was recognized.

Effect of the Invention :

According to the present invention, it is possible to seize correctly the growth state of the microorganism in the fermenter. It is therefore possible to control automatically the concentration of the residual sugar and the temperature and to increase the yield by approximately 5% over that of the prior method.

**Claims**

1. A method for culturing a microorganism aerobically which comprises monitoring the growth state of the microorganism in a fermentation broth in a fermenter by measuring directly and simultaneously the concentration of the microorganism cells, the concentration of the carbon source, the concentration of ammonium ion and the concentration of the desired product in the said fermentation broth in the said fermenter with a single measuring means, and controlling automatically the temperature, the concentration of carbon source and the concentration of ammonium ion in the said fermentation broth on the basis of the measured values.

2. A method for culturing a microorganism aerobically according to Claim 1, wherein the single measuring means is a near-infrared spectrometer.

3. A method for culturing a microorganism aerobically according to Claim 1 or 2, wherein the fermentation broth is that of an amino acid.

4. A method for culturing a microorganism aerobically according to Claim 1 or 2, wherein the fermentation broth is that of a nucleic acid.

5. An apparatus for culturing a microorganism aerobically which comprises a fermenter, having control devices for the feed rate of raw materials, a control device of temperature and a sampling device connected therewith, and having a near-infrared spectrometer connected with the sampling device, and comprising a personal computer which is connected with the near-infrared spectrometer and instructs each control devices to maintain the conditions of the fermentation process at suitable values for the growth of the microorganism.

# Fig. 1

SETTING VALUE OF PRESSURE

SETTING VALUE OF FEED QUANTITY

CONTROL DEVICE OF FEED QUANTITY

FEED SOLUTION

SETTING VALUE OF A VENTILATION FLOW

CONTROL DEVICE OF VENTILATION

FLOWMETER

AIR

CONTROL DEVICE OF PRESSURE

EVACUATION

CONTROL DEVICE OF pH

SETTING VALUE OF pH

NH₃

pH SENSOR

SETTING VALUE OF TEMPERATURE

TEMPERATURE SENSOR

CONTROL DEVICE OF TEMPERATURE

PERSONAL COMPUTER

WATER FOR COOLING

ON-LINE SAMPLING DEVICE

NEAR-INFRARED SPECTROMETER

EP 0 661 380 A2

# Fig. 2

EP 0 661 380 A2

Fig. 3

(o) CONCENTRATION OF MICROORGANISM CELLS (g/ℓ)

(Δ) CONCENTRATION OF CARBON SOURCE (g/dℓ)

(□) CONCENTRATION OF GLUTAMIC ACID (g/dℓ)

CULTURE TIME (hour)

EP 0 661 380 A2

Fig. 4

Fig. 5